(19) Europäisches
Patentamt

European
Patent Office

Office européen
des brevets

(11)  **EP 2 570 123 A1**

(12)  **EUROPEAN PATENT APPLICATION**

(43) Date of publication:
 **20.03.2013  Bulletin 2013/12**

(21) Application number: **12191946.8**

(22) Date of filing: **17.11.2008**

(51) Int Cl.:
*A61K 31/055* (2006.01)   *A61K 31/05* (2006.01)
*A61K 31/167* (2006.01)   *A61K 31/245* (2006.01)
*A61K 31/4425* (2006.01)  *A61K 31/505* (2006.01)
*A61P 31/04* (2006.01)    *A61P 23/02* (2006.01)
*A61P 23/00* (2006.01)

(84) Designated Contracting States:
 **AT BE BG CH CY CZ DE DK EE ES FI FR GB GR
 HR HU IE IS IT LI LT LU LV MC MT NL NO PL PT
 RO SE SI SK TR**

(30) Priority: **15.11.2007  GB 0722349**

(62) Document number(s) of the earlier application(s) in
 accordance with Art. 76 EPC:
 **08850320.6 / 2 219 631**

(71) Applicant: **Reckitt Benckiser Healthcare (UK)
 Limited
 Slough, Berkshire SL1 3UH (GB)**

(72) Inventors:
 • **Haesler Gertrud
  30625 Hannover (DE)**

• **Leuwer, Martin
 Liverpool, Merseyside L69 3BX (GB)**
• **Shephard, Adrian
 Slough, Berkshire SL1 3UH (GB)**
• **Stillings, Michael
 York, Yorkshire YO26 4ZP (GB)**

(74) Representative: **Hodgetts, Catherine Dawn et al
 Reckitt Benckiser
 Corporate Services Limited
 Legal Department - Patents Group
 Dansom Lane
 Hull
 HU8 7DS (GB)**

Remarks:
 This application was filed on 09-11-2011 as a
 divisional application to the application mentioned
 under INID code 62.

(54)  **Use of 2,4-dichlorobenzyl alcohol having an anaesthetic effect**

(57)   The present invention relates to the use of 2,4-dichlorobenzyl alcohol for the preparation of a medicament wherein the 2,4-dichlorobenzyl alcohol exhibits an anaesthetic effect.

EP 2 570 123 A1

**Description**

The present invention relates to the use of anti-bacterial compounds to achieve an anaesthetic effect. In particular, the present invention is directed to the use of 2,4-dichlorobenzyl alcohol (DCBA), amylmetacresol (AMC), cetyl pyridinium chloride (CPC) and hexetidine (HT) to achieve an anaesthetic effect.

2,4-Dichlororbenzyl alcohol (DCBA) is a well-known anti-bacterial compound, and is used against a number of gram positive and gram negative bacteria. 2,4-DCBA has been used with a number of other anti-bacterial agents, e.g. phenolic antiseptics such as amylmetacresol.

EP 0 161 898 teaches the use of compositions containing 2,4-DCBA for the treatment of plaque. US 4 167 583 discloses antimicrobial composition which contain DCBA. The compositions are used to sterilize surgical equipment. WO92/1811 discloses the use of antimicrobial agents, including 2,4-DCBA, in preparations for the treatment of gastric disorders caused by *Helicobacter* pylori. WO96/32934 teaches the use of 2,4-DCBA and amylmetacresol for the preparation of a medicament for the treatment of HIV. WO2005/067906 teaches the use of the same composition for the treatment of severe acute respiratory syndrome (SARS). US 6 251 371 teaches the use of DCBA for the treatment of inflammation of skin or mucosa.

US2007/0202177 teaches the use of amylmetacresol, in an anti-microbial composition. The composition is used for catheter locking solutions or catheter coatings to reduce or prevent infection. WO2005/123074 and WO2004/105758 disclose compositions containing triprolidine for the treatment of sleep disorders. The compositions can contain a further active which includes AMC and DCBA.

Cetylpyridinium chloride (CPC) is commonly used in oral rinses for cleaning mouth and treating minor throat or mouth infections and teething problems. It has also been used as an ingredient in certain pesticides.

Hexetidine is also used in oral rinses for cleaning mouth and treating minor throat or mouth infections.

Most sore throats are caused by viruses, such as cold and flu viruses. Some sore throats can be caused by infection by bacteria, most commonly streptococcus. In extreme cases a course of antibiotics is often required. Viral sore throats, on the other hand, do not respond to antibiotics, and therefore management of the symptoms is required until the virus has been eliminated by the body.

Throat lozenges generally contain at least one of the following ingredients, anaesthetics, painkillers, antibacterials, demulcents.

Typical anaesthetics include benzocaine and lidocaine hydrochloride. Benzydamine hydrochloride and ambroxol also have local anaesthetic properties. Local anaesthetics numb the area they are in contact with and provide temporary relief. Lidocaine hydrochloride and benzocaine are used widely in medical and dental practice for locally numbing the mouth and throat for minor surgical procedures or when a tube must be inserted into the windpipe.

Topically delivered painkillers (or analgesics) include benzydamine hydrochloride, flurbiprofen. These painkillers belong to the group known as NSAIDs (non-steroidal anti-inflammatory drugs) that help reduce pain and swelling. Systemically acting NSAIDS and other pain killers such as paracetamol are also used in the relief of pain. Anaesthesia is generally considered to be the loss or absence of sensation or feeling. The term is commonly used to describe a reversible process which allows operations and painful or unpleasant procedures to be performed without distress to the patient. In contrast analgesia refers to drugs which temporarily relieve or abolish pain. Unlike local anaesthetics, they produce no sensory or motor blockade stopping the activity in the sensory or motor nerves respectively that supply a part of the body.

Typical antibacterials include amylmetacresol, cetylpyridinium chloride, hexetidine and dichlorobenzyl alcohol.

Other known ingredients include volatile oils such as menthol/peppermint, eucalyptus (which can have a sensorial effect) and pectin.

According to a first aspect of the present invention there is provided the use of one or more anti-bacterial compounds for the preparation of a medicament wherein the one or more anti-bacterial compounds exhibits an anaesthetic effect.

Typically the one or more anti-bacterial compounds can be selected from 2,4-dichlorobenzyl alcohol, amylmetacresol, cetylpyridinium chloride, hexetidine, lidocaine, hexylresorcinol and benzocaine. The one or more compounds may be a combination of 2,4-dichlorobenzyl alcohol, amylmetacresol, cetylpyridinium chloride, hexetidine, lidocaine and benzocaine. Preferred combinations can be selected from the group consisting of 2,4-dichlorobenzyl alcohol/amylmetacresol, 2,4-dichlorobenzyl alcohol/cetylpyridinium chloride, 2,4-dichlorobenzyl alcohol/hexetidine, 2,4-dichlorobenzyl alcohol/lidocaine, 2,4-dichlorobenzyl alcohol/benzocaine, amylmetacresol/cetylpyridinium chloride, amylinetacresol/hexetidine, amylmetacresol/lidocaine, amylinetacresol/benzocaine, cetylpyridinium chloride/hexetidine, cetylpyridinium chloride/lidocaine, cetylpyridinium chloride/benzocaine, hexetidine/lidocaine, hexetidine/benzo caine, 2,4-dichlorobenzyl alcohol/amylmetacresol/cetylpyridinium chloride, 2,4-dichlorobenzyl alcohol/amylmetacresol/hexetidine, 2,4-dichlorobenzyl alcohol/amylmetacresol/lidocaine and 2,4-dichlorobenzyl alcohol/amylmetacresol/benzocaine, hexylresorcinol/amylmetacresol, hexylresorcinol/2,4-dichlorobenzyl alcohol, hexylresorcinol/cetylpyridinium chloride, hexylresorcinol/hexetidine, hexylresorcinol/lidocaine, and hexylresorcinol/benzocaine.

The medicament can be in the form of a lozenge, gel, spray, capsule, pastille, gum, dissolving granules, gargle, drink, liquid-shots, tablet, or any other suitable format. The medicament can be in the form of a lozenge.

The base of the medicament which contains the one or more anti-bacterial agents comprises one or more components selected from sucrose, glucose, isomalt, maltitol, xylitol, mannitol.

The medicament can be used to treat sore throats including dry, scratchy, inflamed, swollen, stabbing, burning and painful throats. The medicament can also be used to treat other areas of the mouth such as the gums or tonsils.

The local anaesthetic effect of the medicament can be obtained in a period of less than 10 minutes. The local anaesthetic effect of the medicament can be obtained in a period of less than 5 minutes.

According to a second aspect of the present invention there is provided a method of obtaining a local anaesthetic effect in a patient comprising administering a composition comprising one or more anti-bacterial compounds which exhibits an anaesthetic effect.

Typically the one or more anti-bacterial compounds are selected from 2,4-dichlorobenzyl alcohol, amylmetacresol, cetylpyridinium chloride, hexetidine, lidocaine, hexylresorcinol and benzocaine. The one or more compounds may be a combination of 2,4-dichlorobenzyl alcohol, amylmetacresol, cetylpyridinium chloride, hexetidine, lidocaine and benzocaine. Preferred combinations can be selected from the group consisting of 2,4-dichlorobenzyl alcohol/amylmetacresol, 2,4-dichlorobenzyl alcohol/cetylpyridinium chloride, 2,4-dichlorobenzyl alcohol/hexetidine, 2,4-dichlorobenzyl alcohol/lidocaine, 2,4-dichlorobenzyl alcohol/benzocaine, amylmetacresol/cetylpyridinium chloride, amylmetacresol/ hexetidine, amylmetacresol/lidocaine, amylmetacresol/benzocaine, cetylpyridinium chloride/hexetidine, cetylpyridinium chloride/lidocaine, cetylpyridinium chloride/benzocaine, hexetidine/lidocaine, hexetidine/benzo caine, 2,4-dichlorobenzyl alcohol/amylmetacresol/cetylpyridinium chloride, 2,4-dichlorobenzyl alcohol/amylmetacresol/hexetidine, 2,4-dichlorobenzyl alcohol/amylmetacresol/lidocaine and 2,4-dichlorobenzyl alcohol/amylmetacresol/benzocaine, hexylresorcinol/ amylmetacresol, hexylresorcinol/2,4-dichlorobenzyl alcohol, hexylresorcinol/cetylpyridinium chloride, hexylresorcinol/ hexetidine, hexylresorcinol/lidocaine, and hexylresorcinol/benzocaine.

The local anaesthetic effect can be obtained in a period of less than 10 minutes. The local anaesthetic effect can be obtained in a period of less than 5 minutes.

The patient can be suffering from a sore throat, including dry, scratchy, inflamed, swollen, stabbing, burning and painful throats. The patient may also be suffering from infection of other areas of the mouth such as the gums or tonsils.

Embodiments of the invention will now be described, by way of example only, with reference to the accompanying Figures in which:

Figure 1 illustrates the concentration-response curve for amylmetacresol (AMC);

Figure 2 illustrates the increase in binding affinity with fast inactivation- voltage-dependence of block by amylinetacresol (AMC);

Figure 3 illustrates representative current traces for the concentration- and voltage-dependent increase in blocking potency of amylinetacresol (AMC);

Figure 4 illustrates the concentration-response curve for dichlorobenzylalcohol (DCBA);

Figure 5 illustrates the increase in binding affinity with fast inactivation- voltage-dependence of block by dichlorobenzyl alcohol (DCBA);

Figure 6 illustrates the effects of a combination of amylinetacresol (AMC) and dichlorobenzyl alcohol (DCBA);

Figure 7 illustrates the fast inactivation of an AMC/DCBA combination;

Figure 8 illustrates a concentration response curve for cetylpyridinium chloride (CPC);

Figure 9 illustrates the fast inactivation of cetylpyridinium chloride (CPC);

Figure 10 illustrates representative fast-inactivation current traces for cetylpyridinium chloride (CPC);

Figure 11 illustrates the concentration response curve for hexetidine (HT);

Figure 12 illustrates the fast-inactivation effects of hexetidine (HT);

Figure 13 illustrates representative fast-inactivation current traces for hexetidine (HT);

Figures 14 - 16 illustrate estimated affinities of amylinetacresol (AMC), dichlorobenzyl alcohol (DCBA) and hexetidine (HT) to inactivated channels;

Figure 17 illustrates the concentration-response curve for Hexylresorcinol (HR);

Figure 18 illustrates the concentration dependent increase in binding affinity with fast inactivation: voltage dependence of sodium channel block by Hexylresorcinol (HR); and

Figure 19 illustrates the fast inactivation current traces for hexylresorcinol (HR).

*Transfection and cell culture*

Stably transfected HEK 293 cell lines expressing the $\alpha$-subunit of $NaV_{1.2}$ neuronal sodium channels from a rat were used. The expression vector pRc/CMV (Invitrogen, San Diego, USA) was used for mammalian transfection. Transfection was performed using calcium phosphate precipitation. Permanent expression was achieved by selection for resistance to the aminoglycoside antibiotic geneticin G418 (Life Technology, Eggenstein, Germany). Successful channel expression was verified electrophysiologically.

*Solutions*

Dichlorobenzylalcohol (DCBA), amylmetacresol (AMC), cetylpyridinium chloride (CPC), hexylresorcinol (HR) and hexetidine (HT) were prepared as 1 M stock solution in ethanol prior to the experiments. The stock solution was then dissolved directly in bath solution. The bath solution contained [mM] NaCl 140, $MgCl_2$ 1.0, KCl 4.0, $CaCl_2$ 2.0, Hepes 5.0, dextrose 5.0. Patch electrodes contained [mM] $CsCl_2$ 130, $MgCl_2$ 2.0, EGTA 5.0, Hepes 10. All solutions were adjusted to 290 mosm $1^{-1}$ by the addition of mannitol and to pH 7.4 by the addition ofCsOH.

*Electrophysiology*

Standard whole-cell voltage-clamp experiments were performed at 20°C. Each experiment consisted of test recordings with the drug present at only one concentration, and of drug-free control recordings before and after the test. Each cell was exposed to one test concentration only. At least three or five independent experiments were performed at each concentration depending on the active being tested.

For data acquisition and further analysis, a EPC9 digitally-controlled amplifier was used in combination with Pulse and Pulse Fit software (HEKA Electronics, Lambrecht, Germany). The EPC9 provides automatic subtraction of capacitive and leakage currents by means of a prepulse protocol. The data were filtered at 10 kHz and digitized at 20 $\mu$s per point. The input resistance of the patch pipettes was 2.0-3.5 M$\Omega$ and the capacitances of the cells were 9-15 pF; the residual series resistance (after 50% compensation) was 1.2-2.5 M$\Omega$. Experiments with a rise in series resistance were rejected. To minimize time-dependent shifts in the voltage-dependence of steady-state inactivation, all test experiments were performed within 5 min of patch rupture. Under these experimental conditions, time-dependent hyperpolarizing shifts in control conditions were less than -2 mV.

Drug effects on the peak current amplitude were assessed at a hyperpolarized membrane potentials (-100 mV and -150mV), at a holding potential close to the normal resting potential in physiological conditions (-70 mV), and, additionally, at -100 mV in the presence of a fraction of slow inactivated channels. Slow inactivation was induced by long (2.5 s) prepulses to -90, -70, -50, -30 and -10 mV, followed by a short hyperpolarization to allow recovery from fast-inactivation. In our protocol the recovery interval was prolonged to 100 ms (instead of 10 ms originally described) in order to allow sufficient time not only for drug-free, but also for all drug-bound states to recover from fast inactivation. The residual sodium current ($I_{norm}$) in the presence of a drug (with respect to the peak current elicited with the same protocol during wash-out) elicited with a single test pulse to 0 mV was plotted against the applied concentration of the drug [C]. Concentration-response-curves were fitted using a Hill equation yielding the concentrations for half-maximum channel blockade ($ICR_{50}$), and the respective Hill coefficients $n_H$.

$$(1) \quad \frac{I}{I_{\max}} = \frac{IC_{50}{}^{n_H}}{IC_{50}{}^{n_H} + C^{n_H}}$$

$$(2) \quad \frac{I}{I_{\max}} = a \frac{ICR_{50}^{n_H}}{ICR_{50}^{n_H} + C^{n_H}} + (1-a) \frac{ICI_{50}^{n_H}}{ICI_{50}^{n_H} + C^{n_H}}$$

In the presence of Hexetidine, a 2-state Hill equation, Eq. 2, was applied, yielding the concentrations for half-maximum channel blockade in the inactivated state ($ICI_{50}$), the respective Hill coefficient $n_H$, and the respective amplitudes a and (1-a). The values previously obtained for $ICR_{50}$ and $n_H$ were entered as constant factors.

Drug binding to inactivated channels is hardly accessible to a direct experimental approach, because inactivated channel states and all drug-bound channel states are unavailable on depolarization. A model-dependent method is generally applied to determine the affinity to fast-inactivated channel states. The voltage-dependence of the block and the affinity for fast-inactivated channel states was further assessed by applying a double-pulse protocol. Currents elicited by test pulses ($I_{test}$), starting from varying prepulse potentials (from -150 mV to -5 mV), normalized to the current elicited at the most hyperpolarized prepotential (-150 mV), represent the relative fraction of channels that have not been inactivated during the 100 ms inactivating prepulse. Boltzmann fits to the resulting current-voltage plots yield the membrane potential at half-maximum channel availability ($V_{0.5}$) and the slope factor k, reflecting the 'voltage sensitivity' of inactivation gating (see Eq. 3).

$$(3) \quad \frac{I}{I_{\max}} = \frac{1}{1 + e^{\left(\frac{V_{test} - V_{0.5}}{k}\right)}}$$

In control conditions, the parameters of the Boltzmann fits reflect the voltage-dependence of the distribution between resting and fast-inactivated channels (the availability curve). In the presence of the drug, the steady-state availability curve reflects drug association with rested and fast-inactivated channels, respectively. Tighter binding of the drug to fast-inactivated channels compared to resting channels, a typical feature of sodium channel blockade by lidocaine-like local anesthetics, is revealed by a negative voltage shift $\Delta V_{0.5}$ in the channel availability curve that shows concentration-dependence. The underlying assumption is that the higher amount of channel blockade achieved with consecutive membrane depolarization is determined by the distribution of channels between resting and fast-inactivated states along the voltage axis (i.e. the channel availability curve), and by the different binding affinities of the blocking drug for the two channel states. The voltage shift $\Delta V_{0.5}$ in the presence of a drug would depend upon the drug concentration [C] according to Eq. 4. A fit of the model to $\Delta V_{0.5}$ plotted against concentration yields estimates for the concentration for half-maximum effect on fast-inactivated channels $ICI_{50}$ and the Hill-type coefficient for drug binding to inactivated channels n:

$$(4) \quad \Delta V_{0.5} = k.\ln[(1 + \frac{[C]}{ICR_{50}})/(1 + \frac{[C]^n}{ICI_{50}^n})]$$

K and $ICR_{50}$ are entered as constant factors. K is the slope factor of the availability curve in the controls and $ICR_{50}$ is the concentration for half-maximum effect on resting channels, obtained at -100 mV holding potential.

The accumulation of block during trains of depolarizing pulses suggests that the interval between pulses is too short to allow recovery of sodium channel availability. Use-dependent block was defined as the additional reduction in inward current for the last pulse relative to the first pulse in a test train in the presence of drug assessed at -100 and -70 mV holding potential using trains of 10 ms pulses applied at 10 Hz.

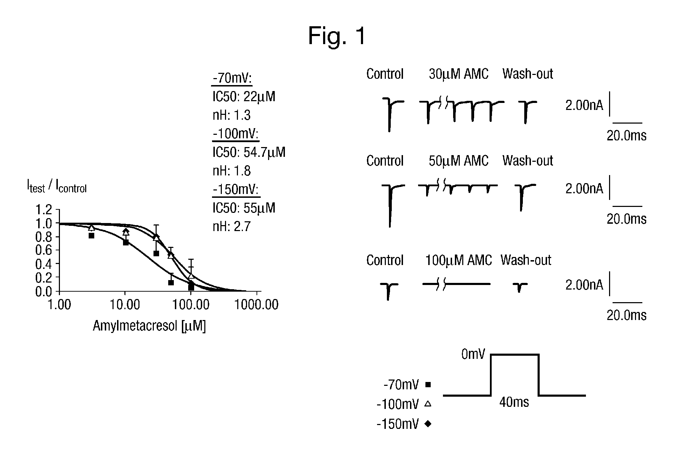

Figure 1 illustrates the concentration-response curve for amylmetacresol (AMC). The left side of Figure 1 illustrates the results obtained when the concentration dependent block of sodium channels was tested with depolarizing pulses to 0mV from a holding potential of -70 mV (filled squares), -100 mV (empty triangles) and -150 mV (filled rhombs), respectively. The peak current elicited in control experiments with the same protocol was used to normalize currents. Each symbol represents the mean value of the residual sodium currents in the presence of the compound ($I_{norm}$, mean $\pm$SD), derived from at least 3 experiments for each concentration tested. Sensitivity to AMC was increased at -70 mV compared to -100 mV.

The right side of Figure 1 illustrates the representative current traces demonstrating the inhibition of sodium currents at different concentrations of AMC. The traces show the first pulse out of a series of 10 pulses from -70 mV to 0 mV applied at 10 Hz in the absence of drug (control, wash-out) and the first and the last three pulses in the presence of AMC. During wash-out, currents reached 94 $\pm$ 6 % of the control current after application of higher concentrations of AMC (> EC$_{50}$) at -100 mV. Recovery of the peak current amplitude during wash-out was dependent on the holding potential- currents reached only 64 $\pm$ 9 % of the control amplitude at -70 mV. Without wishing to be bound by any theory, this finding suggests that depolarization induces a high-affinity state for AMC which impedes its dissociation from the binding site.

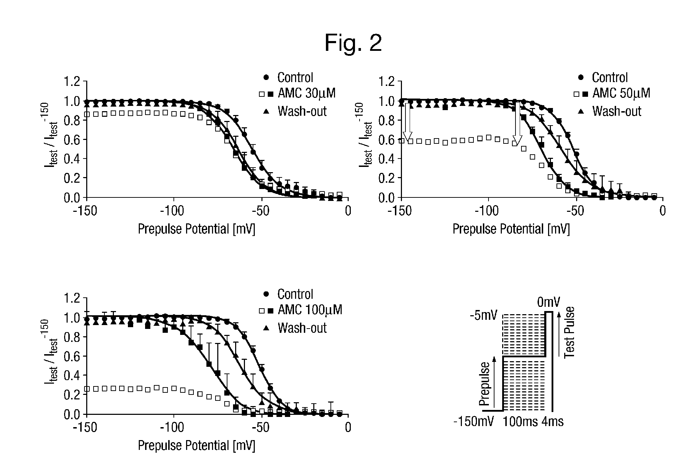

Figure 2 illustrates the increase in binding affinity with fast inactivation- voltage-dependence of block by amylmetacresol (AMC). Steady-state availability curves were assessed by a two pulse protocol in the absence (control: circles; wash-out: triangles) and presence of different concentrations (30, 50, 100 $\mu$M) of Amylmetacresol (squares). Each symbol represents the mean fractional current derived from at least four different experiments, elicited by a 4 ms test pulse to 0 mV, following a 100 ms inactivating prepulse from -150 mV to the indicated prepulse potential. Currents were normalized to maximum value (in each series at -150 mV prepotential), solid lines represent the best Boltzman fit to the data. Test pulse currents were normalized either to maximum value in the presence of drug (filled squares) or to the maximum value in the controls (empty squares). Vertical arrows in the diagram illustrate the increase in peak current suppression induced by 50 $\mu$M AMC at more depolarized potentials versus hyperpolarized prepulse potentials. This reduction in channel availability at depolarized prepotentials resulted in a voltage shift in the midpoints of the availability curve. Concentration-dependence of this effect was used to assess the affinity of inactivated state binding with a model-dependent approach.

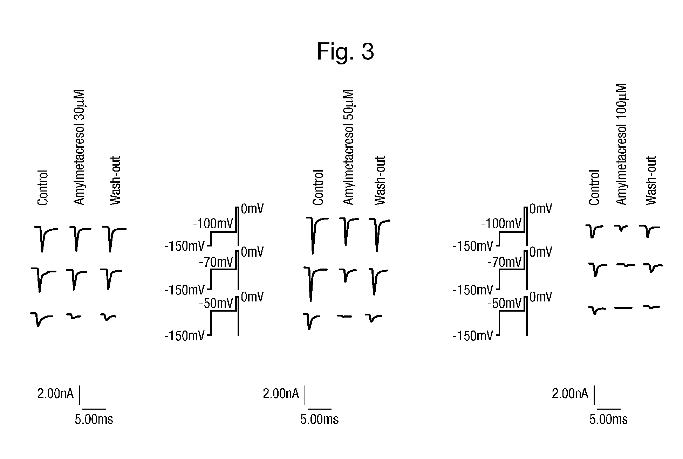

Representative current traces for the concentration- and voltage-dependent increase in blocking potency of Amylmetacresol at depolarized membrane potentials are illustrated in Figure 3. Currents were elicited by application of a 4 ms test pulse to 0mV following a 100 ms prepulse from -150 mV to -100 mV (first column), -70 mV (second column) or -50 mV (third column of traces).Recovery of the peak current amplitude during wash-out reached 90 $\pm$ 3, 91 $\pm$ 3 and 64 $\pm$ 9 % of the control current at -150, -100, and -70 mV holding potential, respectively.

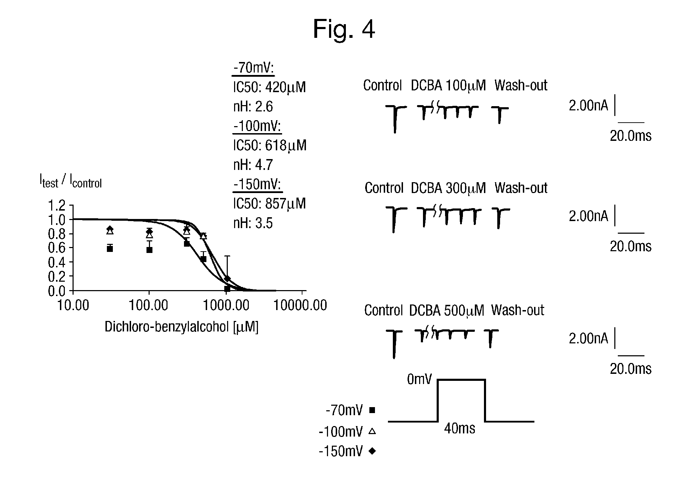

Figure 4 illustrates the concentration-response curve for dichlorobenzyl alcohol (DCBA). The concentration-dependent inhibition of sodium inward currents by dichlorobenzyl alcohol at -70, -100 and -150 mV holding potential, respectively, are shown. Depolarizing pulses to 0 mV were started from either -150 mV (filled rhombs), -100 mV (empty triangles), or -70 mV (filled squares). Currents were normalized to the peak current elicited with the same protocol in the respective control experiment. Each symbol represents the mean value of the residual sodium currents in the presence of drug (I$_{norm}$, mean $\pm$ SD), derived from at least four independent experiments for each concentration tested. Sensitivity was slightly increased at -70 mV holding potential compared to -100 and -150 mV. The solid lines are Hill fits (Eq. 1) to the data with the indicated parameters. Wash-out of the effects of DCBA was incomplete. At -100 mV holding potential, currents reached 71 $\pm$ 3 % of the control current amplitude during wash-out after 300 and 500 $\mu$M DCBA.

At -70 mV, current amplitudes returned to 63 $\pm$ 9 % of the control current during wash-out. The representative current traces on the right illustrate current inhibition by DCBA showing the first pulse out of a series of 10 pulses form -70 to 0 mV applied at 10 Hz during control and wash-out, and the first pulse and the last three pulses in the presence of DCBA.

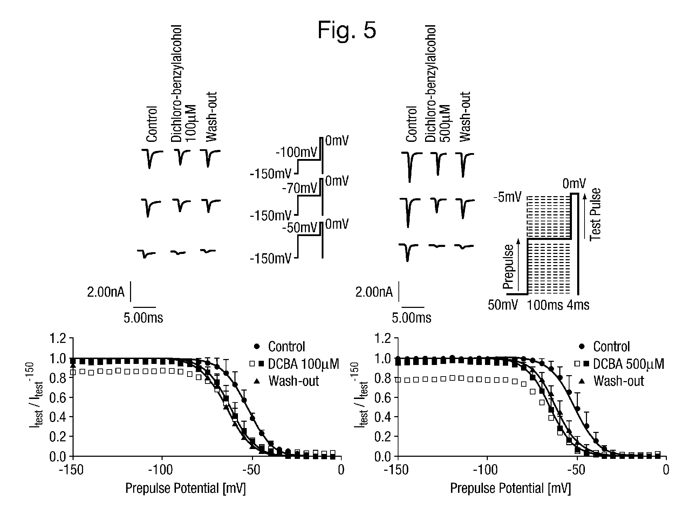

Figure 5 illustrates the increase in binding affinity with fast inactivation- voltage-dependence of block by dichlorobenzyl alcohol (DCBA). The top of Figure 5 illustrates the increase in blocking potency at depolarized membrane potentials for DCBA. Representative current traces in the control and in 100 $\mu$M (left) or 500 $\mu$M dichlorobenzyl alcohol (right) during short (4 ms) test pulses to 0 mV, following a 100 ms prepulse from -150 mV to either -100 mV (1st row of traces), -70 mV (2nd row of traces) or -50 mV (3rd row of traces), which is a membrane potential close to the potential for half-maximum channel inactivation in the controls. Currents returned to 88 $\pm$ 3, 89 $\pm$ 5 and 63 $\pm$ 9 % of the control currents at -150, -100 and -70 mV during wash-out, respectively.

The plots on the bottom of Figure 5 illustrate the results obtained when steady-state availability curves were assessed by a two-pulse protocol in the absence (control; circles, wash-out; triangles) and presence of either 100 $\mu$M (left diagram) or 500 $\mu$M Dichloro-benzylalcohol (right diagram, squares). Each symbol represents the mean fractional current derived from at least four different experiments, elicited by a 4 ms test pulse to 0 mV, following a 100 ms inactivating prepulse from -150 mV to the indicated prepulse potential. Currents were normalized to maximum value (in each series at -150 mV prepotential); solid lines represent the best Boltzmann fit (Eq. 3, methods section) to the data. Test pulse currents in the presence of drug were normalized either to maximum value in the presence of drug (filled symbols) or to maximum value in the controls (empty symbols).

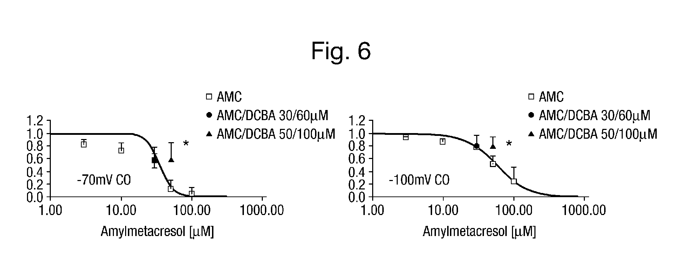

Figure 6 illustrates the effects of a combination of amylmetacresol (AMC) and dichlorobenzyl alcohol (DCBA). Concentration dependent block of sodium channels was tested adding 60 and 100$\mu$M DCBA to 30 and 50 $\mu$M AMC, respectively, at -70 (left) and -100 mV (right) holding potential using the protocols previously described. Each symbol represents the mean value of residual sodium currents (with respect to control) in the presence of AMC alone (empty squares) or in presence of 30 $\mu$M AMC and 60 $\mu$M DCBA (filled circle) or 50 $\mu$M AMC and 100 $\mu$M DCBA (filled triangle), derived from at least 3 experiments for each concentration tested.

A t-test revealed no significant difference comparing the 30 $\mu$M/60 $\mu$M combination of AMC/DCBA to the effect of 30 $\mu$M AMC alone. In the combination with 100 $\mu$M DCBA, the effect of 50 $\mu$M AMC was significantly reduced with respect to the effect of 50 μM AMC alone. Interestingly, there was also no significant difference between the 30 μM/60 μM and the 50 μM/100 μM combination of these two compounds- showing that addition of higher concentrations of DCBA alters the steepness of the concentration-response to AMC.

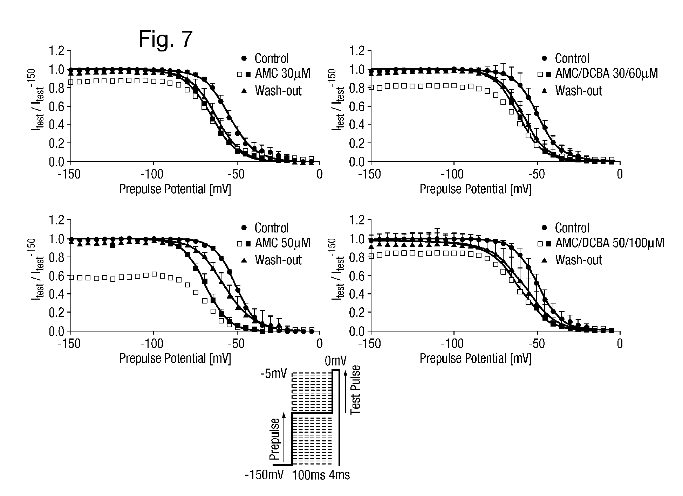

Figure 7 illustrates the fast inactivation of a combination of AMC & DCBA. Steady-state availability curves were assessed by a two pulse protocol in the absence (control: circles; wash-out: triangles) and presence of different concentrations (30 μM/60 μM, 50 μM/100 μM) of the AMC/DCBA-combination (squares). Each symbol represents the mean fractional current derived from at least six different experiments, elicited by a 4 ms test pulse to 0 mV, following a 100 ms inactivating prepulse from -150 mV to the indicated prepulse potential. Currents were normalized to maximum value (in each series at -150 mV prepotential), solid lines represent the best Boltzman fit to the data. Test pulse currents were normalized either to maximum value in the presence of drug combination (filled squares) or to the maximum value in the controls (empty squares).

The effect of the 30 μM/60 μM combination was very similar to the effect of 30 μM AMC alone (top), whereas the effect of the 50 μM/100 μM combination was significantly decreased by the addition of DCBA compared to the effect of 50 μM AMC alone (bottom).

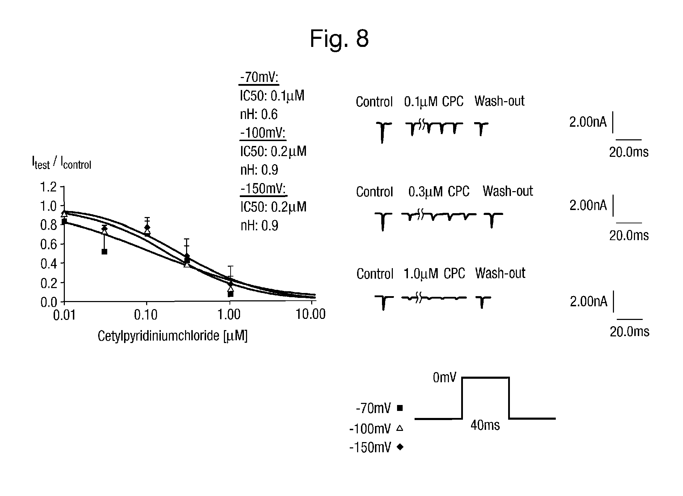

Figure 8 illustrates a concentration response curve for cetylpyridinium chloride (CPC). The left side of Figure 8 illustrates the results obtained when the concentration dependent block of sodium channels was tested with depolarizing pulses to 0 mV from a holding potential of -70 mV (filled squares), -100 mV (empty triangles) and -150 mV (filled rhombs), respectively. The peak current elicited in control experiments with the respective protocol was used to normalize currents. Each symbol represents the mean value of residual sodium currents in presence of the compound ($I_{norm}$, mean $\pm$ SD), derived from at least 5 experiments for each concentration tested. In contrast to HR and AMC, sensitivity to CPC was not increased at -70 mV compared to -100 mV. This finding suggests that CPC binding is not increased in the presence of inactivated channels.

The right side of Figure 8 illustrates representative current traces demonstrating the inhibition of sodium current at different concentrations of CPC. The traces show the first pulse out of a series of 10 pulses from -70 mV to 0 mV applied at 10 Hz in the absence of drug (control, wash-out) and the first and the last three pulses in the presence of CPC. Currents reached 66 $\pm$ 7, 78 $\pm$ 5 and 81 $\pm$ 5 % of control currents during wash- out at -150, -100, and -70 mV holding potential, respectively.

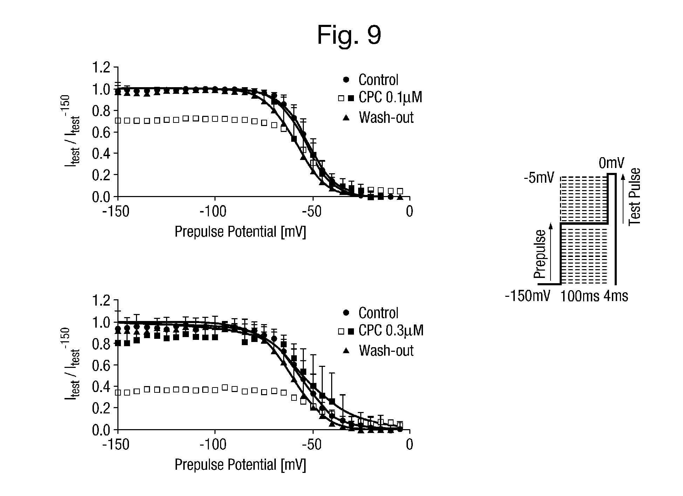

Figure 9 illustrates the fast inactivation of cetylpyridinium chloride (CPC). Steady-state availability curves assessed by a two-pulse protocol in the absence (control: circles; wash-out: triangles) and presence of 0.1 μM (top) and 0.3 μM (bottom) Cetylpyridiniumchloride (squares). Each symbol represents the mean fractional current derived from at least eight different experiments, elicited by a 4 ms test pulse to 0 mV, following a 100 ms inactivating prepulse from -150 mV to the indicated prepulse potential. Currents were normalized to maximum value (in each series at -150 mV prepotential); solid lines represent the best Boltzmann fit (Eq. 3, methods section) to the data. Test pulse currents in the presence of drug were normalized either to maximum value in the presence of drug (filled symbols) or to maximum value in the controls (empty symbols).

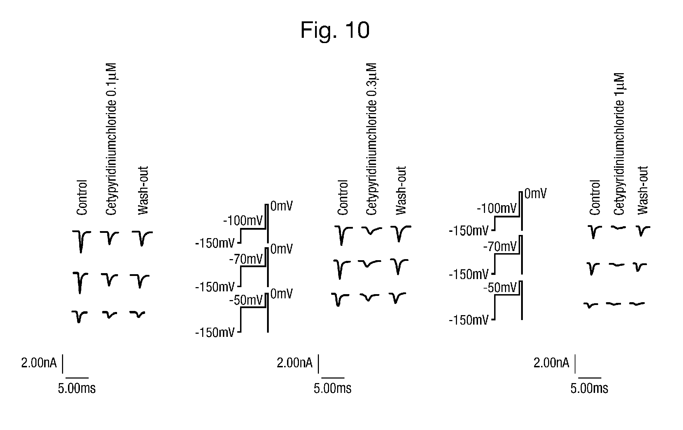

Figure 10 illustrates representative current traces in the absence (control, washout) and presence of 0.1 μM, 0.3 μM and 1 μM CPC, elicited by applying a 4 ms test pulse to 0 mV, following a 100ms prepulse from -150 mV to either -100 mV (first column), -70 mV (second column) or -50 mV (third column). 1 μM CPC induced an almost complete block of sodium inward current, the 0.3 μM concentration is near the $EC_{50}$ for block of resting channels.

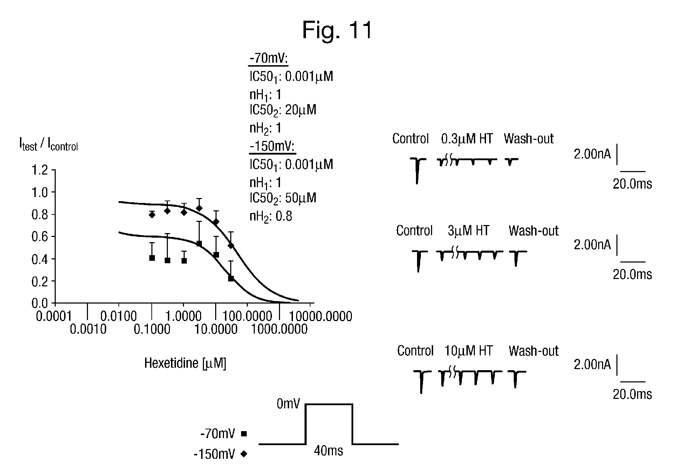

Figure 11 illustrates the concentration response curve for hexetidine (HT). The left side of Figure 11 illustrates concentration-dependent inhibition of sodium inward currents by hexetidine at -70 and -150 mV holding potential, respectively. Depolarizing pulses to 0 mV were started from either -150 mV (filled rhombs) or -70 mV (filled squares). Currents were normalized to the peak current elicited with the same protocol in the respective control experiment. Each symbol represents the mean value of the residual sodium currents in the presence of drug ($I_{norm}$, mean $\pm$ SD), derived from at least four independent experiments for each concentration tested. Sensitivity was increased at -70 mV holding potential compared to -150 mV. At -70 mV, block induced by very low concentration of hexetidine (0.1 μM) was already at 41 $\pm$ 13 % of the control current amplitude. The hexetidine concentration-effect curve was biphasic- the effect of the 0.1 μM concentration did only minimally increase with increasing concentrations up to 1 μM, at concentrations >3 μM, concentration-dependence was steep. The biphasic concentration-effect curve of hexetidine was best described fitting a two-stage Hill equation (Eq. 2) to the averaged data (solid line) with the indicated parameters.

Concentrations above 30 μM were toxic for the seal in whole cell experiments. Thus, although in 3 experiments 100 μM hexetidine induced a complete block of sodium inward currents, this cannot be attributed solely to a specific sodium channel blocking effect.

The representative current traces on the right side of Figure 11 illustrate current inhibition by HT showing the first pulse out of a series of 10 pulses form -70 to 0 mV applied at 10 Hz during control and wash-out, and the first pulse and the last three pulses in the presence of HT.

Recovery of the peak current amplitude during wash-out was dependent on the holding potential. During wash-out, currents reached 47 $\pm$ 11 % of the control current at -70 mV and 80 $\pm$ 8 % at -150 mV.

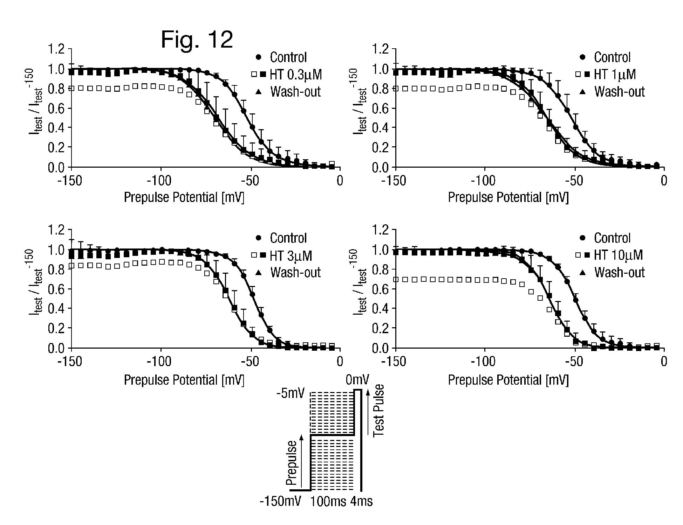

Figure 12 illustrates the fast-inactivation effects of hexetidine (HT). Steady-state availability curves were assessed by a two pulse protocol in the absence (control: circles; wash-out: triangles) and presence of different concentrations (0.3, 1.0, 3.0, 10.0 $\mu$M) of hexetidine (squares). Each symbol represents the mean fractional current derived from at least four different experiments, elicited by a 4 ms test pulse to 0 mV, following a 100 ms inactivating prepulse from -150 mV to the indicated prepulse potential. Currents were normalized to maximum value (in each series at -150 mV prepotential), solid lines represent the best Boltzman fit to the data. Test pulse currents were normalized either to maximum value in the presence of drug (filled squares) or to the maximum value in the controls (empty squares). The voltage shift in the presence of HT shows only very little concentration-dependence over a wide range of concentrations (0.3-3 $\mu$M).

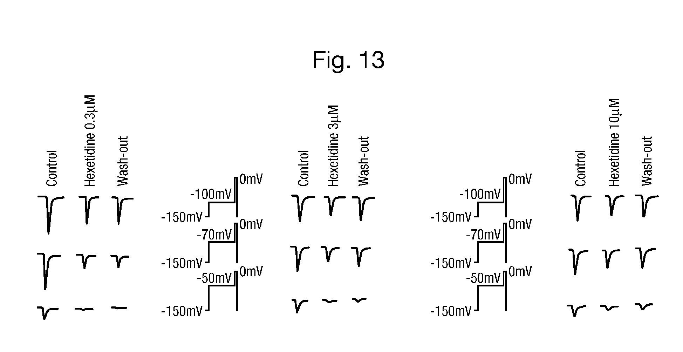

Figure 13 illustrates representative fast-inactivation current traces for the concentration- and voltage-dependent increase in blocking potency of hexetidine at depolarized membrane potentials. Currents were elicited by application of a 4 ms test pulse to 0 mV following a 100 ms prepulse from -150 mV to -100 mV (first row), -70 mV (second row) or -50 mV (row of traces).

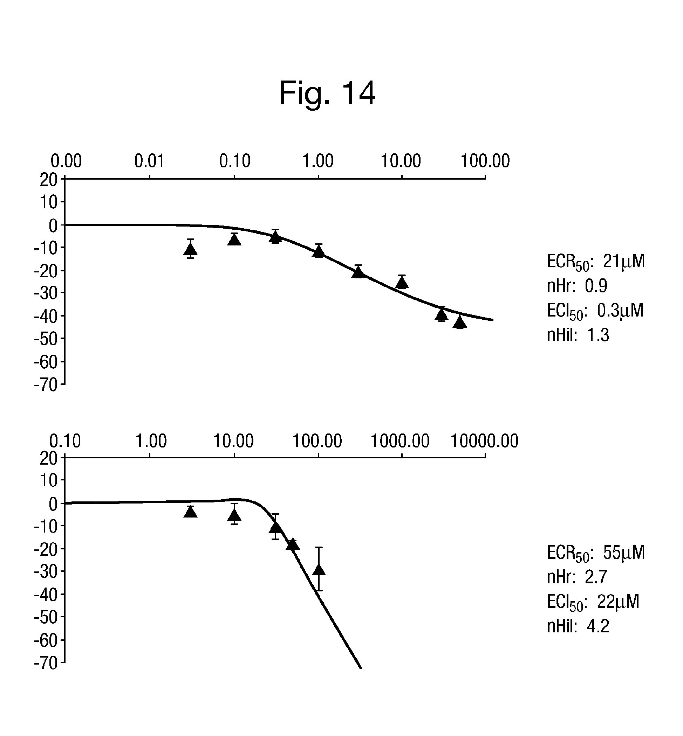

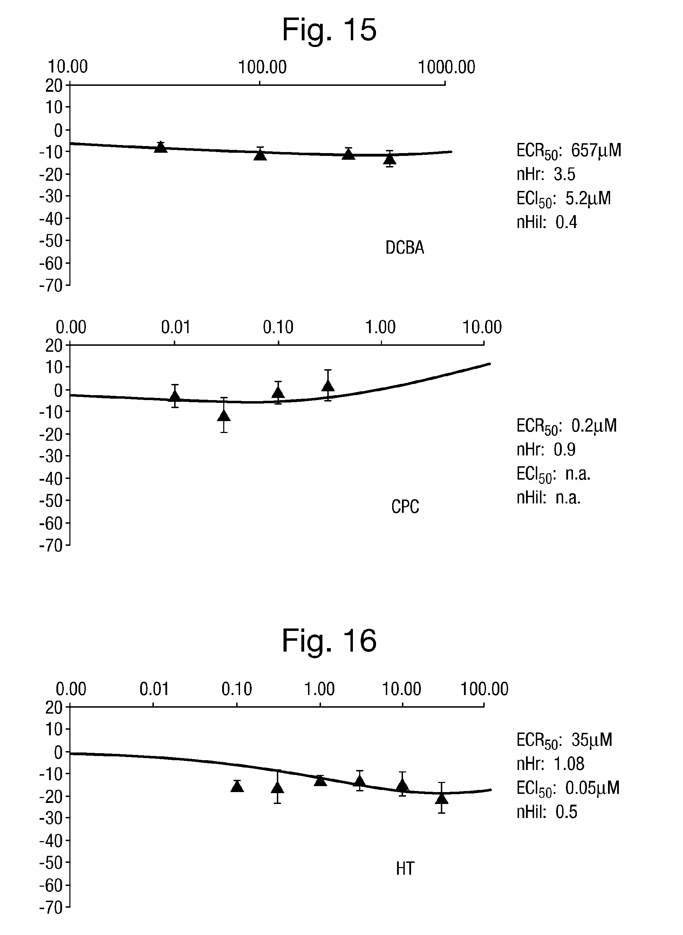

Figures 14 - 16 illustrate estimated affinities of amylmetacresol (AMC), dichlorobenzyl alcohol (DCBA) and hexetidine (HT) to inactivated channels. AMC, HT and DCBA shifted the voltage-dependence of sodium channel availability in the direction of negative potentials indicating that these compounds possess higher binding affinity to inactivated channels. In the case of DCBA and HT, voltage shift showed very little concentration-dependence. The following figures show the concentration-dependence of the drug-induced negative shifts in the midpoints of the steady-state availability plots relative to the starting values in the presence of AMC (Fig. 14), DCBA (top, Fig 15), CPC (bottom, Fig. 15) and HT (Fig. 16). Each symbol represents the mean value derived from at least three different experiments. Error bars are standard deviations; the solid line shows the fit of equation 4 to the averaged data with the indicated parameters. The estimated affinity of AMC, DCBA and HT to inactivated channels was several fold higher compared to resting channels. The voltage shift in the presence of AMC showed a steep concentration-dependence revealed by an estimated Hill coefficient for inactivated state binding of 4.2 and 1.3, respectively. HT and DCBA induced a maximum shift of -21 mV and -15 mV, respectively, which showed little concentration-dependence revealed by Hill coefficients for inactivated state binding of 0.5 and 0.4, respectively. This finding suggests that binding of these compounds in the presence of channel inactivation shows negative cooperativity.

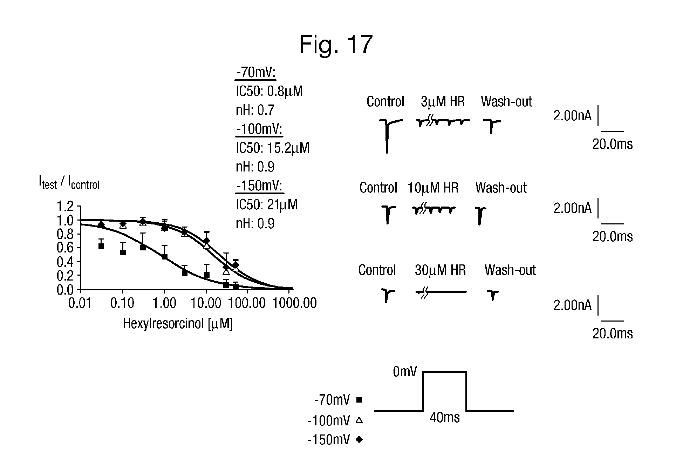

The left side of Figure 17 illustrates the concentration dependent block of sodium channels was tested with depolarizing pulses to 0mV from a holding potential of -70 mV (filled squares), -100 mV (empty triangles), and -150 mV (filled rhombs), respectively. The mean value of the peak current elicited in control experiments with the same protocol was used to normalize currents. Each symbol represents the mean value of the residual sodium currents in the presence of the compound ($I_{norm}$, mean $\pm$SD), derived from at least 5 experiments for each concentration tested, the solid line is a Hill fit to the data with the indicated parameters. Block induced by Hexylresorcinol is clearly voltage-dependent with increased sensitivity at -70 mV holding potential compared to -100 and -150 mV, respectively. Block induced by HR was incomplete even in high concentrations at hyperpolarized holding potentials.

The right side of Figure 17 illustrates representative current traces demonstrating the inhibition of sodium currents at different concentrations of Hexylresorcinol. The traces show the first pulse out of a series of 10 pulses from -70 mV to 0 mV applied at 10 Hz in the absence of drug (control, wash-out) and the first and the last three pulses in the presence of HR. During wash-out, currents reached 90 $\pm$ 10 % of the control current after application of higher concentrations of HR (> $EC_{50}$) at -100 mV. Interestingly, recovery of the peak current amplitude during wash-out was voltage-dependent. Currents reached only around 61 $\pm$ 5% of the control amplitude at -70 mV. This finding might suggest that depolarization induces a high-affinity state for HR which impedes its dissociation from the binding site.

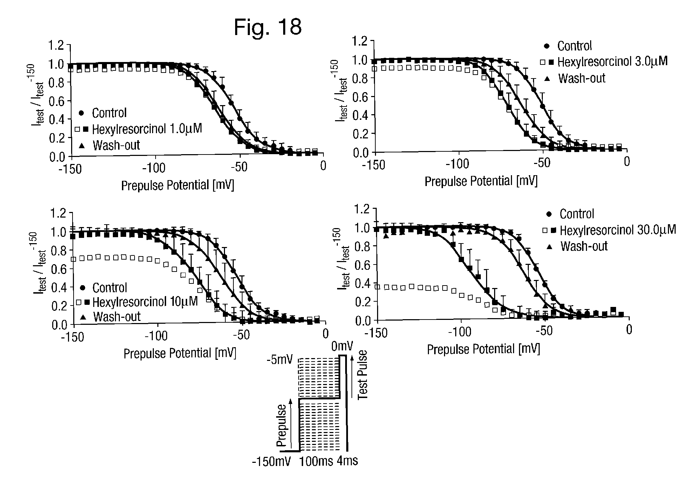

Figure 18 illustrates the steady-state availability curves assessed by a two pulse protocol in the absence (control: circles; wash-out: triangles) and presence of different concentrations (1, 3, 10, 30 $\mu$M) of Hexylresorcinol (squares). Each symbol represents the mean fractional current derived from at least four different experiments, elicited by a 4 ms test pulse to 0 mV, following a 100 ms inactivating prepulse from -150 mV to the indicated prepulse potential. Currents were normalized to maximum value (in each series at -150 mV prepotential), solid lines represent the best Boltzmann fit to the data. Test pulse currents were normalized either to maximum value in the presence of drug (filled squares) or to the maximum value in the controls (empty squares). Block induced by Hexylresorcinol is strongly increased by membrane depolarization. Current inhibition by 1 and 3 $\mu$M Hexylresorcinol is minimal at hyperpolarized holding potentials (between -150 and -85 mV), and is increased to almost complete current inhibition near the potential for half-maximum current inactivation in $Na_v1.2$ (-55 mV). This reduction in channel availability at depolarized prepotentials resulted in a voltage shift in the midpoints of the availability curve (see Fig. 14). Concentration-dependence of this effect was used to assess the affinity to the inactivated state using the model-dependent approach described above (for comparison see Figs. 14, 15 and 16).

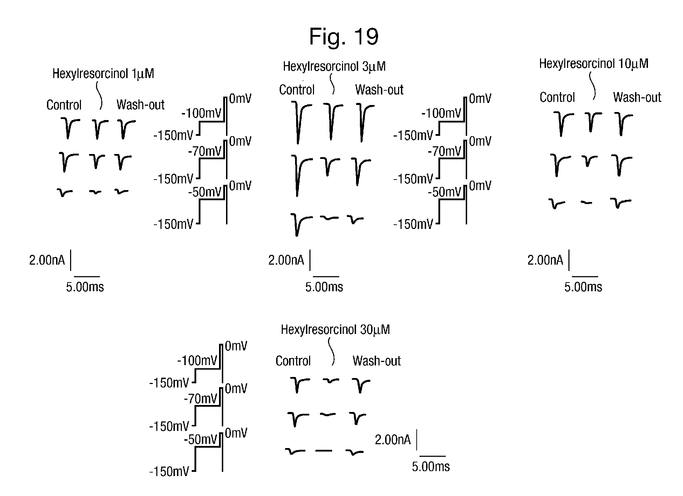

Figure 19 illustrates representative current traces for the voltage - dependent blocking effects of HR in the absence (control, washout) and presence of 1 $\mu$M, 3 $\mu$M, 10 $\mu$M and 30 $\mu$M HR, elicited by applying a 4 ms test pulse to 0 mV, following a 100 ms prepulse from -150 mV to either -100 mV (first row of traces), -70 mV (second row of traces) or -50 mV (third row of traces). Currents reached 95 $\pm$ 3, 94 $\pm$ 4 and 61 $\pm$ 5 % of control currents during wash- out at -150, -100, and -70 mV holding potential, respectively.

Table 1: Half maximum blocking concentrations and Hill coefficients for resting and inactivated state binding

**Table 1**

| Drug | $ECR_{50}$ | $ECI_{50}$ | $nH_R$ | $nH_I$ |
|------|------------|------------|--------|--------|
| AMC | 55$\mu$M | 22$\mu$M | 2.7 | 4.2 |
| DCBA | 657$\mu$M | 5.2$\mu$M | 3.5 | 0.4 |
| CPC | 0.2$\mu$M | N/A | 0.9 | N/A |
| HR | 21 $\mu$M | 0.3 $\mu$M | 0.9 | 1.3 |
| HT | 35 $\mu$M | 0.05$\mu$M | 1.08 | 0.5 |

ECR50 and nH assessed by fitting Eq.3 to the concentration-response plots at -150 mV holding potential. ECI50 and nHi are estimates for the respective effects on inactivated channels derived from a model fit of Eq.4 to the concentration-dependence of the voltage shift in the availability curve.

The blocking effects of amylmetacresol, dichlorobenzyl alcohol, cetylpyridinium chloride, hexylresorcinol and hexetidine on heterologously expressed voltage-gated neuronal sodium channels have been investigated. All compounds blocked depolarization-induced sodium inward currents. Of all compounds studied, cetylpyridinium chloride showed the highest potency to block sodium inward current upon depolarization from hyperpolarized potentials. 1 $\mu$M cetylpyridinium chloride induced almost complete block of sodium inward current. The half maximum resting-state blocking concentration was 0.22 $\mu$M with a Hill coefficient nH of around 1.27. The results show that block induced by cetylpyridinium chloride is concentration, but not voltage-dependent (Figure 11, 12, 13).

Amylmetacresol, dichlorobenzyl alcohol and hexetidine block sodium inward currents in a voltage-and-concentration-dependent manner. Dichlorobenzyl alcohol possessed the lowest potency for blockade of sodium currents of all compounds studied, comparable to the blocking potency of the local anaesthetic lidocaine. The half maximum resting-state blocking concentration was 657 $\mu$M with a steep concentration-dependence at hyperpolarized potentials, and a low concentration-dependence at depolarized potentials. Plotting the shift in the voltage-dependence of inactivation against the applied concentration of dichlorobenzyl alcohol and hexetidine revealed only very little concentration-dependence of the shift. These findings suggest that transition into the inactivated state inhibits binding of additional molecules of dichlorobenzyl alcohol and hexetidine or, alternatively, that dichlorobenzyl alcohol and hexetidine binding inhibit transition into the inactivated channel state.

Furthemore, in the case of hexetidine, a biphasic concentration-response curve with little increase in the blocking effect with increasing concentration in the low concentration range was observed, and a steep concentration-response curve in the range between 3 and 100 $\mu$M HT (EC$_{50}$ 35 $\mu$M). This is consistent with the existence of two distinct binding sites for hexetidine with negative cooperativity for the high-affinity binding site (estimated EC$_{50}$ 0.001 $\mu$M).

Amylmetacresol and hexylresorcinol are the compounds which most resemble lidocaine-like local anaesthetics with respect to higher binding affinity to inactivated channels than to resting channels. The EC$_{50}$ for block of sodium channels upon depolarization from the resting state for amylmetacresol was 55 $\mu$M - thus, around 10-fold higher than the respective potency of lidocaine. A block induced by amylmetacresol was also voltage-dependent and increased with depolarized prepotentials revealed by a shift in the voltage-dependence of inactivation. For amylmetacresol, the estimated ECI$_{50}$ for binding to inactivated channels was 22 $\mu$M, thus, around 3-fold lower than the ECR$_{50}$. The concentration-dependence for both effects- block of resting channels and shift in the voltage-dependence of inactivation- was steep revealed by Hill coefficient n$_H$ around 4.2 for Amylmetacresol. Reduced reversibility of the effect of both drugs at depolarized potentials suggests that drug dissociation from the inactivated-blocked state is less likely than drug dissociation from the resting-blocked state.

The blocking potency of a combination of amylmetacresol and dichlorobenzyl alcohol was investigated in an effort to confirm that addition of dichlorobenzyl alcohol to amylmetacresol increases the potency of amylmetacresol. The results did not show an increase in blocking potency of 30 $\mu$M amylmetacresol by addition of 60 $\mu$M dichlorobenzyl alcohol, and even a decrease in blocking potency of 50 $\mu$M amylmetacresol by addition of 100 $\mu$M dichlorobenzyl alcohol (Figures 6, 7). This finding suggests that dichlorobenzyl alcohol competes with amylmetacresol for the same binding site, ultimately leading to a smaller effect due to the lower potency of dichlorobenzyl alcohol compared to amylmetacresol. Without being bound by any theory, it is believed that the combination of both drugs leads to a ceiling effect of the local anaesthetic effect of amylmetacresol.

Block induced by cetylpyridinium chloride is not voltage-dependent- in contrast to the effects of class Ib lidocaine-like sodium channel blockers. Among the compounds studied, amylmetacresol is around 10-fold more potent than lidocaine. Amylmetacresol is comparable to class Ib lidocaine-like sodium channel blocking drugs showing the typical pattern of tighter binding to inactivated channel states. Dichlorobenzyl alcohol is about as potent as lidocaine with respect to block of resting channels. However, in contrast to lidocaine, transition into the inactivated state impedes binding of further dichlorobenzyl alcohol molecules. Addition of dichlorobenzyl alcohol to amylmetacresol decreases the effect of amylmetacresol applied as the sole drug- this observation can be explained assuming that both compounds compete for the same binding site. A combination of both drugs ensures that the local anaesthetic effect of amylmetacresol is attenuated at higher concentrations.

The biphasic concentration-response curve for hexetidine led to the conclusion that there exists two distinct binding sites for this compound.

Further modifications and improvements can be incorporated without departing from the scope of the invention disclosed herein.

**Claims**

1. The use of 2,4-dichlorobenzyl alcohol for the preparation of a medicament wherein the one or more anti-bacterial compounds exhibits an anaesthetic effect.

2. The use as claimed in Claim 1 wherein the medicament further comprises in combination with said 2,4-dichlorobenzyl alcohol one or more anti-bacterial compounds selected from amylmetacresol, cetylpyridinium chloride, hexetidine, and hexylresorcinol.

3. The use as claimed in Claim 2 wherein the combination is selected from the group consisting of 2,4-dichlorobenzyl alcohol/amylmetacresol, 2,4-dichlorobenzyl alcohol/cetylpyridinium chloride, 2,4-dichlorobenzyl alcohol/hexetidine, 2,4-dichlorobenzyl alcohol/lidocaine, 2,4-dichlorobenzyl alcohol/benzocaine, 2,4-dichlorobenzyl alcohol/amylmetacresol/cetylpyridinium chloride, 2,4-dichlorobenzyl alcohol/amylmetacresol/hexetidine, 2,4-dichlorobenzyl alcohol/amylmetacresol/lidocaine, 2,4-dichlorobenzyl alcohol/amylmetacresol/benzocaine and hexylresorcinol/2,4-dichlorobenzyl alcohol.

4. The use as claimed in any of the preceding Claims wherein the medicament is in the form of a lozenge, gel, spray, capsule, pastille, gum, dissolving granules, gargle, drink, liquid-shots, tablet, or any other suitable form.

5. The use as claimed in Claim 4 wherein the medicament is in the form of a lozenge.

6. The use as claimed in any of the preceding Claims wherein the base of the medicament which contains the one or more anti-bacterial agents comprises one or more components selected from sucrose, glucose, isomalt, maltitol, xylitol, mannitol.

7. The use as claimed in any of the preceding Claims wherein the medicament is used to treat sore throats, including dry, scratchy, inflamed, swollen, stabbing, burning and painful throats, or other areas of the mouth such as the gums or tonsils.

8. A method of obtaining a local anaesthetic effect in a patient comprising administering a composition comprising 2,4-dichlorobenzyl alcohol.

9. The method as claimed in Claim 8 wherein the composition further comprises a combination of 2,4-dichlorobenzyl alcohol and one or more anti-bacterial compounds selected from amylmetacresol, cetylpyridinium chloride, hexetidine, lidocaine, hexylresorcinol and benzocaine.

10. The method as claimed in Claim 9 wherein the combination is selected from the group consisting of 2,4-dichlorobenzyl alcohol/amylmetacresol, 2,4-dichlorobenzyl alcohol/cetylpyridinium chloride, 2,4-dichlorobenzyl alcohol/hexetidine, 2,4-dichlorobenzyl alcohol/lidocaine, 2,4-dichlorobenzyl alcohol/benzocaine, 2,4-dichlorobenzyl alcohol/amylmetacresol/cetylpyridinium chloride, 2,4-dichlorobenzyl alcohol/amylmetacresol/hexetidine, 2,4-dichlorobenzyl alcohol/amylmetacresol/lidocaine, 2,4-dichlorobenzyl alcohol/amylmetacresol/benzocaine and hexylresorcinol/2,4-dichlorobenzyl alcohol.

11. The method as claimed in any of Claims 8 - 10 wherein the patient is suffering from a sore throat including dry, scratchy, inflamed, swollen, stabbing, burning and painful throats, or other areas of the mouth such as the gums or tonsils.

# Fig. 1

-70mV:
IC50: 22μM
nH: 1.3
-100mV:
IC50: 54.7μM
nH: 1.8
-150mV:
IC50: 55μM
nH: 2.7

Control    30μM AMC    Wash-out

2.00nA
20.0ms

Control    50μM AMC    Wash-out

2.00nA
20.0ms

Control    100μM AMC    Wash-out

2.00nA
20.0ms

$I_{test} / I_{control}$

1.2
1.0
0.8
0.6
0.4
0.2
0.0

1.00    10.00    100.00    1000.00

Amylmetacresol [μM]

0mV

-70mV ■
-100mV △
-150mV ◆

40ms

# Fig. 2

EP 2 570 123 A1

Fig. 3

14

## Fig. 4

-70mV:
IC50: 420µM
nH: 2.6
-100mV:
IC50: 618µM
nH: 4.7
-150mV:
IC50: 857µM
nH: 3.5

Control DCBA 100µM Wash-out

2.00nA

20.0ms

Control DCBA 300µM Wash-out

2.00nA

20.0ms

Control DCBA 500µM Wash-out

2.00nA

20.0ms

I_test / I_control

1.2
1.0
0.8
0.6
0.4
0.2
0.0

10.00    100.00    1000.00    10000.00

Dichloro-benzylalcohol [µM]

0mV

-70mV ■
-100mV △
-150mV ◆

40ms

# Fig. 5

# Fig. 6

Fig. 7

## Fig. 8

-70mV:
IC50: 0.1μM
nH: 0.6
-100mV:
IC50: 0.2μM
nH: 0.9
-150mV:
IC50: 0.2μM
nH: 0.9

I_test / I_control

Cetylpyridiniumchloride [μM]

Control   0.1μM CPC   Wash-out

2.00nA

20.0ms

Control   0.3μM CPC   Wash-out

2.00nA

20.0ms

Control   1.0μM CPC   Wash-out

2.00nA

20.0ms

0mV

-70mV ■
-100mV △
-150mV ◆

40ms

## Fig. 9

# Fig. 10

# Fig. 11

-70mV:
$IC50_1$: 0.001μM
$nH_1$: 1
$IC50_2$: 20μM
$nH_2$: 1
-150mV:
$IC50_1$: 0.001μM
$nH_1$: 1
$IC50_2$: 50μM
$nH_2$: 0.8

$I_{test} / I_{control}$

1.2
1.0
0.8
0.6
0.4
0.2
0.0

0.0001    0.0100    1.0000    100.0000    10000.0000
    0.0010    0.1000    10.0000    1000.0000

Hexetidine [μM]

-70mV ■
-150mV ◆

0mV

-70mV ■
-150mV ◆       40ms

Control    0.3μM HT    Wash-out         2.00nA

20.0ms

Control    3μM HT    Wash-out         2.00nA

20.0ms

Control    10μM HT    Wash-out         2.00nA

20.0ms

Fig. 12

# Fig. 13

## Fig. 14

ECR$_{50}$: 21µM
nHr: 0.9
ECl$_{50}$: 0.3µM
nHil: 1.3

ECR$_{50}$: 55µM
nHr: 2.7
ECl$_{50}$: 22µM
nHil: 4.2

# Fig. 15

ECR$_{50}$: 657µM
nHr: 3.5
ECI$_{50}$: 5.2µM
nHil: 0.4

DCBA

ECR$_{50}$: 0.2µM
nHr: 0.9
ECI$_{50}$: n.a.
nHil: n.a.

CPC

# Fig. 16

ECR$_{50}$: 35µM
nHr: 1.08
ECI$_{50}$: 0.05µM
nHil: 0.5

HT

# Fig. 17

-70mV:
IC50: 0.8μM
nH: 0.7
-100mV:
IC50: 15.2μM
nH: 0.9
-150mV:
IC50: 21μM
nH: 0.9

$I_{test}$ / $I_{control}$

Hexylresorcinol [μM]

Control   3μM HR   Wash-out       2.00nA
                                   20.0ms

Control   10μM HR   Wash-out      2.00nA
                                   20.0ms

Control   30μM HR   Wash-out      2.00nA
                                   20.0ms

0mV

-70mV ■
-100mV △
-150mV ◆

40ms

# Fig. 18

## Fig. 19

Europäisches
Patentamt

European
Patent Office

Office européen
des brevets

# EUROPEAN SEARCH REPORT

Application Number

EP 12 19 1946

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/110871 A (NAVEH PHARMA 1996 LTD [IL]; EILAT ERAN [IL]; PRIMOR NITSAN [IL]) 4 October 2007 (2007-10-04) * claim 4 * * page 5, line 16 - line 17 * * page 12, line 5 - line 14 * * page 12, line 19 - line 27 * * abstract * | 1-11 | INV. A61K31/055 A61K31/05 A61K31/167 A61K31/245 A61K31/4425 A61K31/505 A61P31/04 A61P23/02 |
| Y | WO 03/089007 A (ADCOCK INGRAM INTELLECTUAL PRO [ZA]; PARSONS BRADLEY RYAN [ZA]; DU PLE) 30 October 2003 (2003-10-30) * page 2, paragraph 5; claim 6 * * page 4, last paragraph * * claim 6 * | 2,3,9,10 | A61P23/00 |
| Y | DATABASE MEDLINE [Online] US NATIONAL LIBRARY OF MEDICINE (NLM), BETHESDA, MD, US; 2002, KAPIC ELVEDINA ET AL: "[Hexetidine--an oral antiseptic].", XP002691563, Database accession no. NLM11917691 * abstract * & MEDICINSKI ARHIV 2002, vol. 56, no. 1, 2002, pages 43-48, ISSN: 0350-199X | 2-7,9-11 | |
| Y | US 5 753 270 A (BEAUCHAMP PATRICK A [CA] ET AL) 19 May 1998 (1998-05-19) * claim 26 * | 2-7,9-11 | |
| Y | US 6 565 832 B1 (HASLWANTER JOSEPH A [US] ET AL) 20 May 2003 (2003-05-20) * column 3, line 36 - line 37 * | 2-7,9-11 | |

TECHNICAL FIELDS SEARCHED (IPC)

A61K

-/--

The present search report has been drawn up for all claims

| Place of search | Date of completion of the search | Examiner |
|---|---|---|
| The Hague | 5 February 2013 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 12 19 1946

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 4 917 894 A (MATTHIAS JOSEPH A [US] ET AL) 17 April 1990 (1990-04-17) <br> * abstract * <br> * claim 1 * <br> ----- | 2-7,9-11 | |
| A | HAESELER G ET AL: "VOLTAGE-DEPENDENT BLOCKADE OF NORMAL AND MUTANT MUSCLE SODIUM CHANNELS BY BENZYLALCOHOL", EUROPEAN JOURNAL OF PHARMACOLOGY, ELSEVIER SCIENCE, NL, vol. 130, no. 6, 1 July 2000 (2000-07-01), pages 1321-1330, XP001023967, ISSN: 0014-2999 <br> * page 1321 * <br> ----- | 1-11 | |
| T | VANESSA BUCHHOLZ ET AL: "Topical antiseptics for the treatment of sore throat block voltage-gated neuronal sodium channels in a local anaesthetic-like manner", NAUNYN-SCHMIEDEBERG'S ARCHIVES OF PHARMACOLOGY, SPRINGER, BERLIN, DE, vol. 380, no. 2, 15 April 2009 (2009-04-15), pages 161-168, XP019702120, ISSN: 1432-1912 <br> * the whole document * <br> ----- | 1-11 | TECHNICAL FIELDS SEARCHED (IPC) |

The present search report has been drawn up for all claims

| Place of search | Date of completion of the search | Examiner |
|---|---|---|
| The Hague | 5 February 2013 | Langer, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 570 123 A1**

**ANNEX TO THE EUROPEAN SEARCH REPORT**
**ON EUROPEAN PATENT APPLICATION NO.**

EP 12 19 1946

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2013

| Patent document cited in search report | | Publication date | Patent family member(s) | | Publication date |
|---|---|---|---|---|---|
| WO 2007110871 | A | 04-10-2007 | EP | 2015761 A2 | 21-01-2009 |
| | | | WO | 2007110871 A2 | 04-10-2007 |
| WO 03089007 | A | 30-10-2003 | AU | 2003219348 A1 | 03-11-2003 |
| | | | WO | 03089007 A1 | 30-10-2003 |
| US 5753270 | A | 19-05-1998 | CA | 1273576 A1 | 04-09-1990 |
| | | | DE | 3880769 D1 | 09-06-1993 |
| | | | DE | 3880769 T2 | 11-11-1993 |
| | | | EP | 0308210 A1 | 22-03-1989 |
| | | | JP | 1151522 A | 14-06-1989 |
| | | | JP | 2668800 B2 | 27-10-1997 |
| | | | US | 5753270 A | 19-05-1998 |
| US 6565832 | B1 | 20-05-2003 | CA | 2333442 A1 | 31-07-2001 |
| | | | US | 6565832 B1 | 20-05-2003 |
| | | | US | 2003185763 A1 | 02-10-2003 |
| US 4917894 | A | 17-04-1990 | CA | 1324961 C | 07-12-1993 |
| | | | US | 4917894 A | 17-04-1990 |
| | | | ZA | 8904857 A | 27-02-1991 |

EPO FORM P0459

For more details about this annex : see Official Journal of the European Patent Office, No. 12/82

32

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0161898 A
- US 4167583 A
- WO 921811 A
- WO 9632934 A
- WO 2005067906 A
- US 6251371 B
- US 20070202177 A
- WO 2005123074 A
- WO 2004105758 A